# EUROPÄISCHE PATENTANMELDUNG

(11) **EP 1 366 711 A1**
(43) Veröffentlichungstag der Anmeldung: **03.12.2003**
(21) Anmeldenummer: 02012054.9
(22) Anmeldetag: 31.05.2002
(51) Int. Cl.: A61B 5/15, A61M 5/34

(54) **Adapter für eine Blutentnahmevorrichtung**

(71) Anmelder: KABE Labortechnik Gesellschaft mit beschränkter Haftung, D-51588 Nümbrecht-Elsenroth (DE)
(72) Erfinder: Kolpe, Dieter, 51647 Wiehl (DE)
(74) Vertreter: Christophersen & Partner

(57) **Zusammenfassung**

Vorgeschlagen wird ein Adapter für eine Blutentnahmevorrichtung. Der Adapter (5) ist mit einer an ihrer Innenwandung (39) mit einer Axialführung versehenen Hülse (7) versehen. Um vor dem Hintergrund einer Vielzahl teilweise konkurrierender Blutentnahme-Systeme einen Adapter mit erweiterten Einsatzmöglichkeiten zu schaffen, wird in die Hülse konzentrisch zu deren Innenwandung ein Einsatz (11) in Form eines an beiden Seiten offenen Röhrchens (24) eingesetzt. Das Röhrchen (24) ist an seiner Innenwandung seinerseits mit einer Axialführung für ein in das Röhrchen (24) einsetzbares Blutentnahmeröhrchen versehen.

## Beschreibung

Die Erfindung betrifft einen Adapter für eine Blutentnahmevorrichtung, wobei Bestandteil des Adapters eine an ihrer Innenwandung mit einer Axialführung versehene Hülse ist.

Derartige Adapter für Blutentnahmevorrichtungen sind bekannt. Zur Entnahme von Teilmengen an Blut, zum Beispiel für Laborzwecke, ist z. B. an einen Verbindungsschlauch zwischen Einstechkanüle und Blutbeutel ein Adapter angeschlossen, in den sich dann zur Probenentnahme ein Blutentnahmeröhrchen hineinschieben läßt. Derartige Adapter sowie Blutentnahmeröhrchen sind z. B. aus den Druckschriften DE 196 47 976 A1, DE 30 495 03 C2 oder GB 2 027 168 A bekannt.

Bereits die voranstehend zitierten Druckschriften zeigen eine Mehrzahl unterschiedlicher Systeme, wobei die Unterschiede sowohl in der Dimensionierung der Anschlüsse von Blutentnahmeröhrchen einerseits und Adapter andererseits bestehen, als auch in der Art der Verbindung zwischen diesen beiden Teilen. Am Markt wird darüber hinaus eine Vielzahl weiterer, prinzipiell aber ähnlich aufgebauter Blutentnahmesysteme angeboten. Aus Sicht der Hersteller derartiger Blutentnahmesysteme ist diese Systemvielfalt durchaus erwünscht, wenn nicht sogar angestrebt. Anders stellt sich die Situation auf der Anwenderseite dar, zum Beispiel Kliniken oder Arztpraxen. Hier wäre ein höherer Grad an Kompatibilität der einzelnen Systemkomponenten erwünscht, um es so zum Beispiel dem ärztlichen Personal zu ermöglichen, die Adapter eines Herstellers mit Entnahmeröhrchen eines anderen Herstellers zu kombinieren.

Der Erfindung liegt daher die Aufgabe zugrunde, vor dem Hintergrund einer Vielzahl teilweise konkurrierender Blutentnahme-Systeme einen Adapter mit erweiterten Einsatzmöglichkeiten zu schaffen.

Zur Lösung dieser Aufgabe wird bei einem Adapter der eingangs genannten Art vorgeschlagen, daß in die Hülse konzentrisch zu deren Innenwandung ein Einsatz in Form eines an beiden Enden offenen Röhrchens eingesetzt ist, welches an seiner Innenwandung seinerseits mit einer Axialführung für ein in das Röhrchen einsetzbares Blutentnahmeröhrchen versehen ist.

Kerngedanke der Erfindung ist es daher, den Adapter je nach Einsatzzweck mit oder ohne den Einsatz in Form eines an beiden Enden offenen Röhrchens zu verwenden. Wird die Hülse des Adapters mit dem zusätzlichen Einsatz versehen, ergibt sich eine Aufnahmeöffnung für entweder sehr schlank gestaltete Blutentnahmeröhrchen oder Blutentnahmeröhrchen, die an ihrer Abschlusskappe mit einem relativ schlanken zylindrischen Ansatz versehen sind. Ein solches Blutentnahmeröhrchen ist zum Beispiel aus der DE 30 49 503 C2 bekannt. Andererseits lassen sich, falls der röhrchenförmige Einsatz nicht eingesetzt wird, Blutentnahmeröhrchen mit größerem Anschlussdurchmesser in den Adapter einsetzen. Solche Blutentnahmeröhrchen sind zum Beispiel aus der DE 196 47 976 A1 oder der GB 2 027 168 A bekannt. Bei ihnen weist die als Anschluß dienende Abschlusskappe des Entnahmeröhrchens einen größeren Durchmesser auf, als der Grundkörper des Entnahmeröhrchens.

Gemäß einer bevorzugten Ausgestaltung sind der offene Rand der Hülse einerseits und der Einsatz andererseits mit zueinander korrespondierenden Elementen einer Drehverriegelung versehen. Auf diese Weise läßt sich der Einsatz nach Art eines Bajonetts in den Adapter einsetzen, und darin sicher verriegeln.

Ferner wird vorgeschlagen, daß das hülsenseitige Element der Drehverriegelung mindestens eine Ausnehmung ist, die sich jeweils vom Rand der Hülse bis zu einer Hinterschneidung erstreckt.

Ferner wird vorgeschlagen, daß die einsatzseitigen Elemente der Drehverriegelung mindestens zwei von dem Röhrchen von außen weg führende Stege sind. Vorzugsweise verbinden diese Stege das Röhrchen mit einem Ring, der die Hülse konzentrisch umgibt. Auf diese Weise ist der Einsatz zwecks des Einsetzens in den Adapter oder des Herausnehmens aus dem Adapter besonders gut zwischen zwei Fingern greifbar, was die Handhabung in der täglichen klinischen Praxis erleichtert. Zu diesem Zweck ist es ferner von Vorteil, wenn der Ring auf seiner Außenseite mit einer reibungserhöhenden Riffelung versehen ist.

Die von dem Röhrchen des Einsatzes nach außen weg führenden Stege weisen vorzugsweise jeweils einen Kreisquerschnitt auf.

Eine weitere bevorzugte Ausgestaltung der Blutentnahmevorrichtung ist gekennzeichnet durch einen die Außenseite des Röhrchens über dessen gesamten Umfang umgebenden Flansch, von dessen Außenumfang die Stege nach außen abstehen. Dieser Flansch führt zu einer gesteigerten Festigkeit und Stabilität des Einsatzes gegenüber Verwindungskräften, wie sie beim Einsetzen oder Herausnehmen des Blutentnahmeröhrchens auftreten.

Bei einer weiteren bevorzugten Ausgestaltung ist vorgesehen, daß der Adapter mit einer zentral in die Hülse sowie in das Röhrchen des Einsatzes hineinragenden Kanüle versehen ist, und das Röhrchen mit seinem Abschlussrand weiter in Richtung auf den offenen Rand der Hülse vorsteht, als die Spitze der Kanüle. Auf diese Weise bewirkt der in den Adapter eingesetzte Einsatz einen erhöhten Schutz gegen Verletzungen an der scharfen Kanülenspitze. Da der Durchmesser des Röhrchens geringer ist, als die Dicke des menschlichen Fingers, wird durch den nach außen über die Kanülenspitze vorstehenden Abschlußrand des Röhrchens eine versehentliche Berührung dieser Kanülenspitze sicher verhindert.

Schließlich wird mit einer bevorzugten Ausgestaltung der Blutentnahmevorrichtung vorgeschlagen, daß das Röhrchen des Einsatzes nahe seines Abschlussrandes mit mindestens einem nach außen abstehenden Verriegelungsnocken versehen ist, und das Röhrchen außerdem mindestens eine Ausnehmung aufweist, die sich von dem Abschlussrand bis zu einer Hinterschneidung erstreckt. Auf diese Weise sind bei Verwendung des Einsatzes mindestens zwei derzeit am Markt besonders verbreitete Systeme an Blutentnahmeröhrchen wahlweise in dem Adapter fixierbar.

Weitere Einzelheiten und Vorteile werden nachfolgend anhand von Beispielen und unter Bezugnahme auf die Zeichnungen erläutert. Darin zeigen:
- Fig. 1: In Übersichtsdarstellung eine Blutentnahmevorrichtung einschließlich eines Blutbeutels;
- Fig.2: Den Adapter der Blutentnahmevorrichtung in vergrößerter Schnittdarstellung;
- Fig.3: Eine Seitenansicht des Endes des Adapters;
- Fig.4: Eine der Fig. 3 entsprechende Seitenansicht, jedoch nach Entfernen eines Einsatzes aus dem Adapter;
- Fig.5: In Seitenansicht den Einsatz für den Adapter;
- Fig.6: Eine Draufsicht auf den Einsatz;
- Fig 7a bis 7c: Verschiedene Stadien beim Ansetzen eines Blutentnahmeröhrchens der Blutentnahmevorrichtung an den Adapter;
- Fig.8a bis 8c: Verschiedene Stadien beim Ansetzen eines anders gestalteten Blutentnahmeröhrchens an den Adapter;
- Fig.9: Die Kombination des Adapters mit einem wiederum anders gestalteten Blutentnahmeröhrchen und
- Fig.10: Die Kombination des Adapters mit einem wiederum anders gestalteten Blutentnahmeröhrchen.

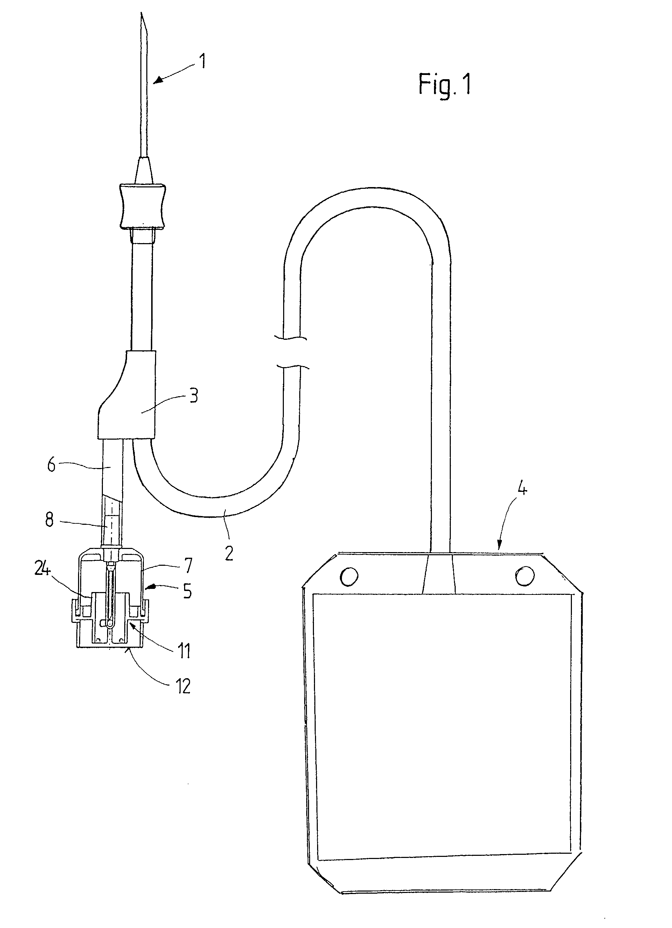

In Figur 1 ist eine Blutentnahmevorrichtung dargestellt, mit einer in die Vene des Patienten einführbaren Einstechkanüle 1, einem Schlauch 2 mit Verzweigungsstück 3, Blutbeutel 4 sowie einem Adapter 5. Der Adapter 5 ist an ein kurzes Schlauchstück 6 angeschlossen, welches an dem Verzweigungsstück 3 von dem Schlauch 2 abzweigt. Der Adapter 5 besteht u.a. aus einer am einen Ende geschlossenen, und am anderen Ende offenen, zylindrisch gestalteten Hülse 7 aus Kunststoff. In dem geschlossenen Ende findet sich eine Öffnung für die Verbindung mit dem Schlauchstück 6. Zur Herstellung dieser Verbindung ist das Ende des Schlauchstücks 6 mit einem Verbindungsstück 8 aus Polycarbonat verklebt. In das Verbindungsstück 8 wiederum greift ein sogenannter Luer-Konus des Adapters 5 ein, wodurch eine flüssigkeitsdichte Verbindung zwischen Schlauchstück 6 und dem Innenraum des Adapters 5 entsteht.

Der Adapter 5 ist in seinem Inneren mit einer an sich bekannten, schräg angeschnittenen Kanüle 9 versehen, die vollständig von einem durchstechbaren Gummischlauch 10 umgeben ist. Einzelheiten betreffend diese Kanüle, den Gummischlauch sowie die Verbindungstechnik beim Ansetzen eines Blutentnahmeröhrchens sind ausführlich zum Beispiel in der DE 196 47 976 A1 erläutert.

In die zylindrisch gestaltete Hülse 7 des Adapters 5 ist ein Einsatz 11, ebenfalls aus Kunststoff eingesetzt. Auf diesen Einsatz wird später noch näher einzugehen sein.

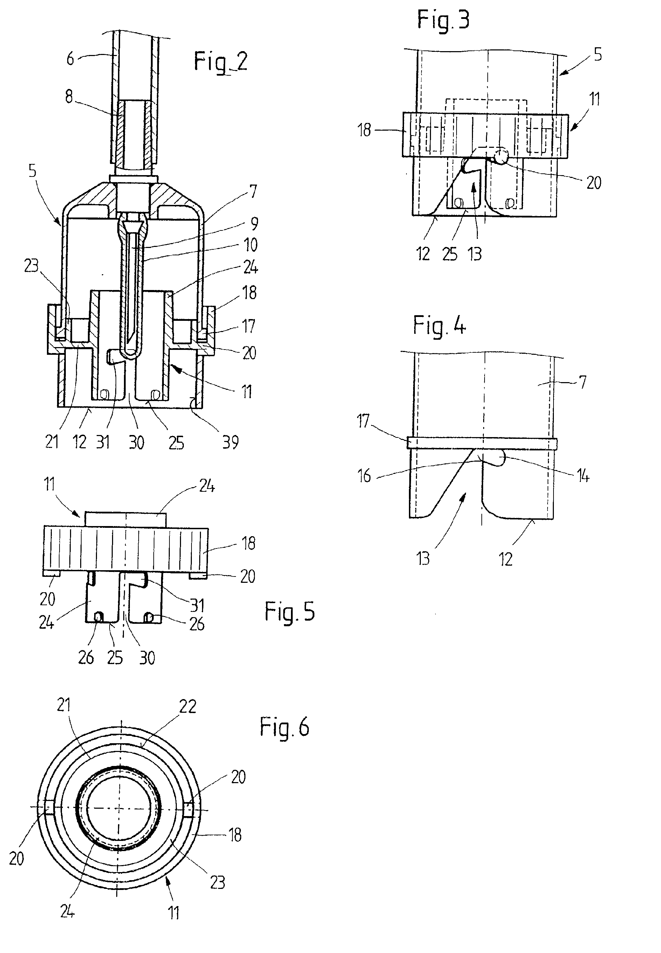

In Figur 4 ist das Ende des Adapters ohne den Einsatz dargestellt. Zu erkennen ist, daß sich vom offenen Rand 12 der Hülse 7 aus Ausnehmungen 13 erstrekken, die in Ansicht etwa trichterförmig gestaltet sind, und sich in Richtung auf den Adapter verjüngen. In dem engsten der Teil der Ausnehmung 13 geht diese in eine Hinterschneidung 14 über, d. h. der äußerste Ort der Ausnehmung 13 hat einen geringeren Abstand zu dem Rand 12 der Hülse, als die Spitze 16 der Hinterschneidung. Die so in dem Wandmaterial der Hülse gestaltete Ausnehmung 13 ist Bestandteil einer Drehverriegelung, zu der ferner korrespondierend gestaltete Verriegelungselemente an entweder dem Einsatz 11 oder einem später noch zu erläuternden Blutentnahmeröhrchen gehören.

Über ihren Umfang betrachtet, ist die Hülse 7 mit insgesamt vier der beschriebenen Ausnehmungen 13 versehen, und zwar jeweils um 90 Grad versetzt zueinander. Oberhalb der Ausnehmungen 13 ist an der Hülse 7 ein umlaufender Bund 17 angeformt.

In Figur 3 ist ebenfalls das Ende des Adapters 5 dargestellt, diesmal jedoch gemeinsam mit dem darin eingesetzten Einsatz 11. Zu erkennen ist, daß der Einsatz 11 über einen Ring 18 verfügt, dessen Durchmesser den Außendurchmesser der Hülse übersteigt, wodurch der Ring 18 die Mantelfläche der Hülse umgibt. An seiner Außenseite ist der Ring 18 des Einsatzes mit einer seine Griffigkeit erhöhenden Riffelung versehen.

Einstückiger Bestandteil des Einsatzes 11 sind Stege 20, welche den Ring 18 mit weiteren, inneren Bestandteilen des Einsatzes 11 verbinden. Beim Ausführungsbeispiel sind insgesamt zwei Stege 20 vorhanden, die einander in Bezug auf die Mittelachse des Einsatzes 11 um 180 Grad gegenüberliegen. Die Stege 20 weisen jeweils einen Kreisquerschnitt auf, sind also nach Art von Nocken geformt. Sie befinden sich exakt in der durch die Mantelfläche der Hülse 7 definierten Ebene, durchdringen also das Material der Hülse 7. Dies ist selbstverständlich nur dort möglich, wo die Hülse 7 mit den in den Hinterschneidungen 14 endenden Ausnehmungen 13 versehen ist. Hierbei ist die Querschnittsfläche der Stege 20 so bemessen, daß diese in diesen Hinterschneidungen 14 einrasten können.

Zur Montage des Einsatzes 11 in dem Adapter 5 wird daher der Einsatz 11 von der offenen Seite über den Rand 12 des Adapters 5 geschoben, wobei die Stege 20 ihren Weg durch die Ausnehmungen 13 hindurch finden. Nachdem die Stege 20 die maximale Tiefe in der Ausnehmung 13 erreicht haben, wird der Einsatz 11 geringfügig gedreht, wodurch jeder Steg 20 in die entsprechende Hinterschneidung 14 gelangt, und dort formschlüssig verriegelt. Damit ist auch der Einsatz 11 unverlierbar an dem Adapter 5 verriegelt.

Die Figuren 5 und 6 lassen am besten die Einzelheiten des Einsatzes 11 erkennen. Dieser besteht aus dem bereits beschriebenen, geriffelten Ring 18, den davon nach innen abstehenden Stegen 20 sowie einem ringförmigen Flansch 21, von dessen Außenumfang 22 die Stege 20 nach außen abstehen. Der Flansch 21 ist, wie insbesondere Figur 2 erkennen läßt, selbst wiederum mit einem Ring 23 versehen, der sich in montiertem Zustand entlang der inneren Mantelfläche der Hülse 7 erstreckt. Der Flansch 21 wiederum steht mit einem Röhrchen 24 in Verbindung, welches den innersten Teil des Einsatzes 11 bildet. Alle vorbeschriebenen Teile, d. h. Röhrchen 24, Flansch 21, Ring 23, Stege 20 sowie äußerer Ring 18 sind einstückig miteinander, und vorzugsweise als Kunststoff-Spritzteil gebildet. Das Röhrchen 24 des Einsatzes 11 ist, wie insbesondere Figur 2 erkennen läßt, in montiertem Zustand konzentrisch in dem Adapter 5 angeordnet. Das Röhrchen 24 erstreckt sich mit seinem Abschlussrand 25 weiter in Richtung auf den offenen Rand 12 der Hülse 7, als die Spitze der Kanüle 9. Da außerdem der Durchmesser des Röhrchens 24 relativ gering ist, verhindert das Röhrchen 24 mit seinem Abschlussrand 25 einen versehentlichen Berührungskontakt mit der scharfen Spitze der Kanüle 9. Der Einsatz 11 erhöht daher die Sicherheit beim Umgang mit dem Adapter 5.

Das Röhrchen 24 des Einsatzes 11 ist mit mehreren verschiedenen Verriegelungsstrukturen versehen. Zunächst sind an der Außenseite des Röhrchens 24 nahe seines Abschlussrandes 25 vier nach außen abstehende Verriegelungsnokken 26 angeformt. Des weiteren ist das Röhrchen 24 mit einer Ausnehmung 30 versehen, die, von dem Abschlussrand 25 ausgehend, bis zu einer Hiinterschneidung 31 führt. Über den Umfang verteilt sind insgesamt vier Ausnehmungen 30 mit Hinterschneidungen 31 vorgesehen. Deren Funktion ist analog der Funktion der Ausnehmungen 13 und Hinterschneidungen 14 der Hülse 7, nämlich die Realisierung des einen Bestandteils einer Drehverriegelung.

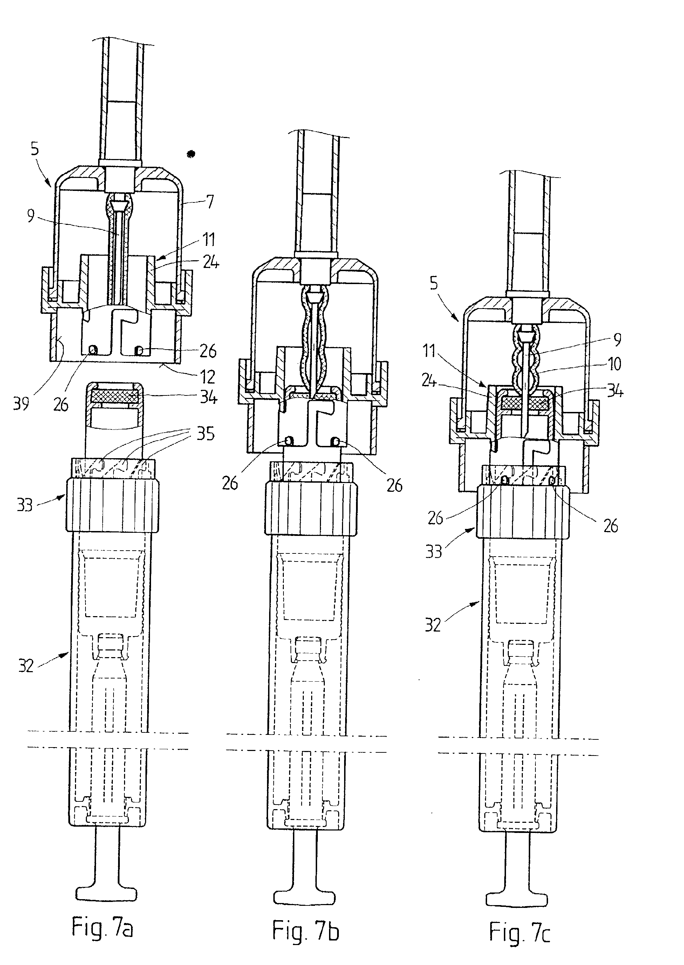

Infolge des wahlweise in den Adapter 5 einsetzbaren Einsatzes lassen sich mehrere am Markt gängige Blutentnahmeröhrchen darin einsetzen. Einige Alternativen werden nachfolgend anhand der Figuren 7a bis 7c, 8a bis 8c, 9 und 10 erläutert.

Die Figuren 7a bis 7c zeigen in drei Stadien das Ansetzen eines Blutentnahmeröhrchens 32. Den Abschluss des Blutentnahmeröhrchens 32 bildet eine Kappe 33, in deren Ende eine von der Kanüle 9 durchstechbare Membran 34 aus Gummi angeordnet ist. Jener Längsabschnitt der Kappe 33, in der sich die Membran 34 befindet, ist relativ schlank ausgebildet. Um diese Kappe aufzunehmen, muß der Adapter 5 daher mit dem Einsatz 11 versehen sein, der wie oben beschrieben an dem Adapter verriegelt wird. Sodann läßt sich das Entnahmeröhrchen, wie dies die Figuren 7b und 7c erkennen lassen, axial in den Adapter hineinschieben, wobei der zylindrische Abschnitt der Kappe 33 in das Röhrchen 24 gelangt. Die Verriegelung erfolgt mittels der Verriegelungsnocken 26 des Röhrchens 24, die hierbei in entsprechend gestaltete Verriegelungsstrukturen 35 an der Kappe 33 gelangen.

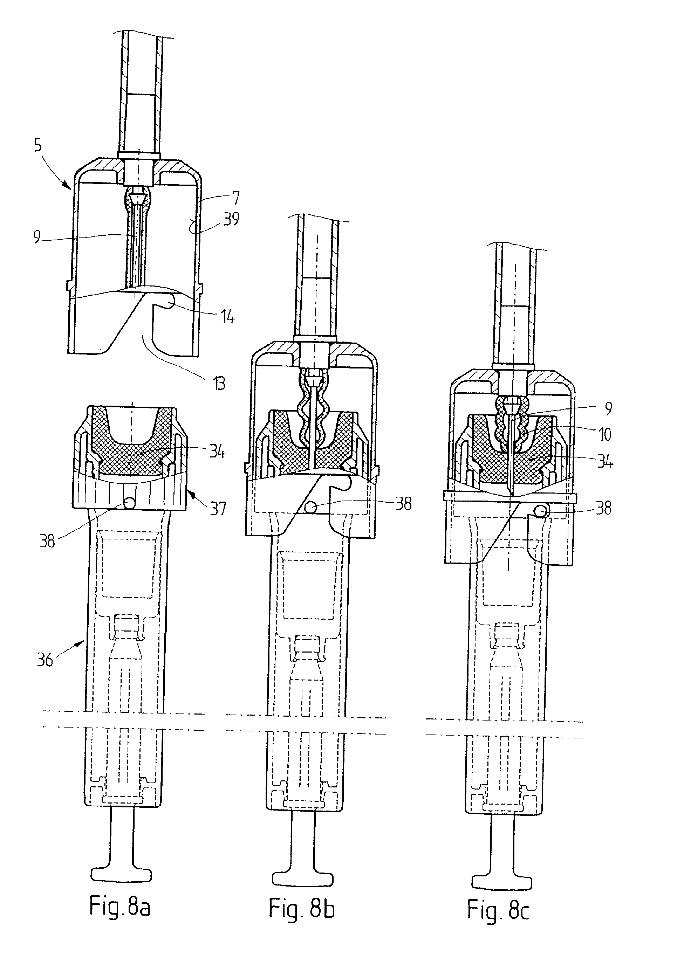

Die Figuren 8a bis 8c zeigen das Ansetzen eines anders gestalteten Blutentnahmeröhrchens 36. Dieses weist eine Kappe 37 mit relativ großem Durchmesser auf. Am unteren Rand der Kappe sind Verriegelungsnocken 38 angeformt. Diese gelangen über die Ausnehmung 13 in die Hinterschneidung 14 der Hülse 7, und werden dort durch Drehen des Entnahmeröhrchens 36 verriegelt. Hierbei wird der Adapter 5 ohne den Einsatz 11 verwendet, die Innenwandung 39 der Hülse 7 dient als Axialführung für das Blutentnahmeröhrchen.

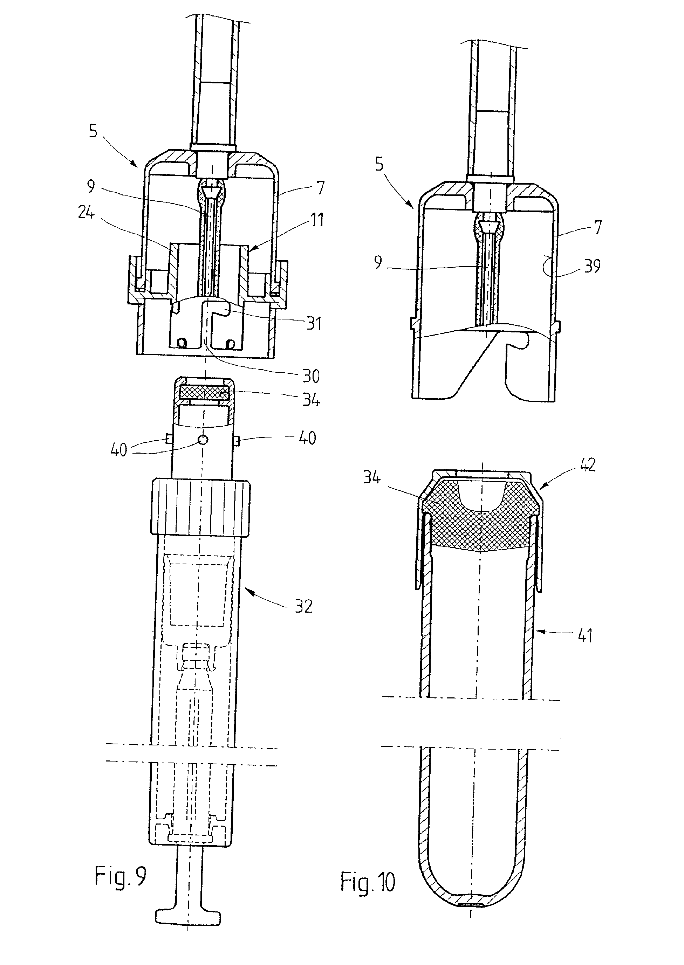

In Figur 9 ist wiederum eine Einsatzmöglichkeit mit in den Adapter 5 eingesetztem Einsatz 11 dargestellt. Das wiederum mit einer schlank auslaufenden Kappe versehene Blutentnahmeröhrchen 32 weist an seiner Kappe Verriegelungsnocken 40 auf. Beim axialen Ansetzen des Blutentnahmeröhrchens gelangen die Verriegelungsnocken 40 entlang der Ausnehmung 30, und durch leichte Drehung in den Bereich der Hinterschneidung 31, womit dann das Blutentnahmeröhrchen in dem Einsatz 11 des Adapters verriegelt ist.

Bei der Ausführungsform nach Figur 10 schließlich wird ein Blutentnahmeröhrchen 41 nach dem Vakuum-Prinzip verwendet. Eine Verriegelung an dem Adapter ist in diesem Fall prinzipbedingt nicht erforderlich. Der Adapter 5 wird ohne den Einsatz verwendet, d. h. die Innenwandung 39 der Hülse 7 dient der Axialführung der Kappe 42 des Blutentnahmeröhrchens 41.

### Bezugszeichenliste

- 1: Einstechkanüle
- 2: Schlauch
- 3: Verzweigungsstück
- 4: Blutbeutel
- 5: Adapter
- 6: Schlauchstück
- 7: Hülse
- 8: Verbindungsstück
- 9: Kanüle
- 10: Gummischlauch
- 11: Einsatz
- 12: Rand
- 13: Ausnehmung
- 14: Hinterschneidung
- 16: Spitze der Hinterschneidung
- 17: Bund
- 18: Ring
- 20: Steg
- 21: Flansch
- 22: Außenumfang
- 23: Ring
- 24: Röhrchen
- 25: Abschlußrand
- 26: Verriegelungsnocken
- 30: Ausnehmung
- 31: Hinterschneidung
- 32: Blutentnahmeröhrchen
- 33: Kappe
- 34: Membran
- 35: Verriegelunsstruktur
- 36: Blutentnahmeröhrchen
- 37: Kappe
- 38: Verriegelungsnocken
- 39: Innenwandung
- 40: Verriegelungsnocken
- 41: Blutentnahmeröhrchen
- 42: Kappe

## Patentansprüche

1. Adapter für eine Blutentnahmevorrichtung, wobei Bestandteil des Adapters eine an ihrer Innenwandung (39) mit einer Axialführung versehene Hülse (7) ist,
**dadurch gekennzeichnet,**
**daß** in die Hülse (7) konzentrisch zu deren Innenwandung (39) ein Einsatz (11) in Form eines an beiden Enden offenen Röhrchens (24) eingesetzt ist, welches an seiner Innenwandung seinerseits mit einer Axialführung für ein in das Röhrchen (24) einsetzbares Blutentnahmeröhrchen (32) versehen ist.

2. Adapter nach Anspruch 1, **dadurch gekennzeichnet, daß** der offene Rand (12) der Hülse (7) einerseits und der Einsatz (11) andererseits mit zueinander korrespondierenden Elementen einer Drehverriegelung versehen sind.

3. Adapter nach Anspruch 2, **dadurch gekennzeichnet, daß** das hülsenseitige Element der Drehverriegelung mindestens eine Ausnehmung (13) ist, wobei sich die Ausnehmung jeweils vom Rand (12) der Hülse (7) bis zu einer Hinterschneidung (14) erstreckt.

4. Adapter nach Anspruch 2, **dadurch gekennzeichnet, daß** die einsatzseitigen Elemente der Drehverriegelung mindestens zwei von dem Röhrchen (24) nach außen weg führende Stege (20) sind.

5. Adapter nach Anspruch 4, **dadurch gekennzeichnet, daß** die Stege (20) das Röhrchen (24) mit einem Ring (18) verbinden, der die Hülse (7) konzentrisch umgibt.

6. Adapter nach Anspruch 5, **dadurch gekennzeichnet, daß** der Ring (18) an seiner Außenseite mit einer reibungserhöhenden Riffelung versehen ist.

7. Adapter nach einem der Ansprüche 4 bis 6, **dadurch gekennzeichnet, daß** die Stege (20) jeweils einen Kreisquerschnitt aufweisen.

8. Adapter nach einem der Ansprüche 4 bis 7, **gekennzeichnet durch** einen die Außenseite des Röhrchens (24) über dessen gesamten Umfang umgebenden Flansch (21), von dessen Außenumfang (22) die Stege (20) nach außen abstehen.

9. Adapter nach einem der vorangehenden Ansprüche, **dadurch gekennzeichnet, daß** der Adapter (5) mit einer zentral in die Hülse (7) sowie in das Röhrchen (24) des Einsatzes (11) hineinragenden Kanüle (9) versehen ist, und daß das Röhrchen (24) mit seinem Abschlussrand (25) weiter in Richtung auf den offenen Rand (12) der Hülse (7) vorsteht, als die Spitze der Kanüle (9).

10. Adapter nach einem der vorhergehenden Ansprüche, **dadurch gekennzeichnet, daß** das Röhrchen (24) des Einsatzes (11) nahe seines Abschlussrandes (25) mit mindestens einem nach außen abstehenden Verriegelungsnocken (26) versehen ist, und daß das Röhrchen (24) außerdem mindestens eine Ausnehmung (30) aufweist, die sich von dem Abschlussrand (25) bis zu einer Hinterschneidung (31) erstreckt.
